# EUROPEAN PATENT APPLICATION

(11) **EP 4 079 367 A1**
(43) Date of publication of application: **26.10.2022**
(21) Application number: 20900879.6
(22) Date of filing: 06.11.2020
(51) Int. Cl.: A61M 25/092, A61M 25/01

(54) **DELIVERY SHEATH AND MEDICAL DEVICE**

(30) Priority: 17.12.2019 CN 201911301098; 17.12.2019 CN 201911300548; 17.12.2019 CN 201911300491
(71) Applicant: Lifetech Scientific (Shenzhen) Co., Ltd., Shenzhen, Guangdong 518000 (CN)
(72) Inventor: LI, Anning, Shenzhen, Guangdong 518000 (CN); HU, Longhu, Shenzhen, Guangdong 518000 (CN); TANG, Huiqiang, Shenzhen, Guangdong 518000 (CN); WANG, Cui, Shenzhen, Guangdong 518000 (CN)
(74) Representative: dompatent von Kreisler Selting Werner - Partnerschaft von Patent- und Rechtsanwälten mbB
(86) International application number: PCT/CN2020/126982
(87) International publication number: WO 2021/120917

(57) **Abstract**

A delivery sheath and a medical device. The delivery sheath includes an outer canal and a traction structure. The outer canal includes a connecting canal, a canal body, and a distal canal. The canal body is located between the connecting canal and the distal canal. The canal body includes a first flexible canal, a spacer canal, and a second flexible canal. The spacer canal is provided between a distal end of the first flexible canal and a proximal end of the second flexible canal. The hardness of the connecting canal is greater than that of the first and second flexible canals, and the hardness of the spacer canal is greater than that of the first and second flexible canals. The traction structure includes a first traction fiber and a second traction fiber.

## Description

### Technical Field

The embodiments relate to the field of medical devices, and, in particular, to a delivery sheath and a medical device.

### Background

Minimally invasive interventional surgery is increasingly favored by doctors and patients due to its less traumatic nature. In minimally invasive surgery, a delivery sheath is used to establish a delivery channel from the outside to the inside of the body (mainly a diseased site), through which an implanted device can be delivered to the diseased site or withdrawn. Drugs can also be injected into the patient's body or body fluid inside the patient's body can be exported through the delivery channel.

Existing delivery sheaths used for delivering a left atrial appendage occluder include pre-shaped delivery sheaths.

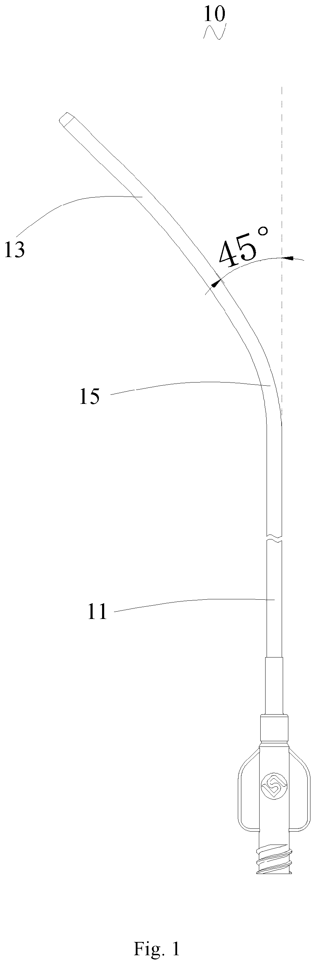

As shown in Fig. 1, the pre-shaped delivery sheath includes a single-bendable pre-shaped delivery sheath 10. In an example, the single-bend pre-shaped delivery sheath 10 includes a proximal section 11, a distal section 13, and a bending section 15. The proximal section 11 and the distal section 13 are straight canals in a natural state. The proximal section 11 is located on one side of a proximal end of the single-bendable pre-shaped delivery sheath 10, and the distal section 13 is located on one side of a distal end of the single-bendable pre-shaped delivery sheath 10. The bending section 15 is of a canal structure that is of a bent shape in a natural state, and its number is one section. The bending section 15 is located between the proximal section 11 and the distal section 13, and the proximal section 11 and the distal section 13 are connected through the bending section 15. An extending direction of the proximal section 11 and an extending direction of the distal section 13 form an included angle of 45 degrees.

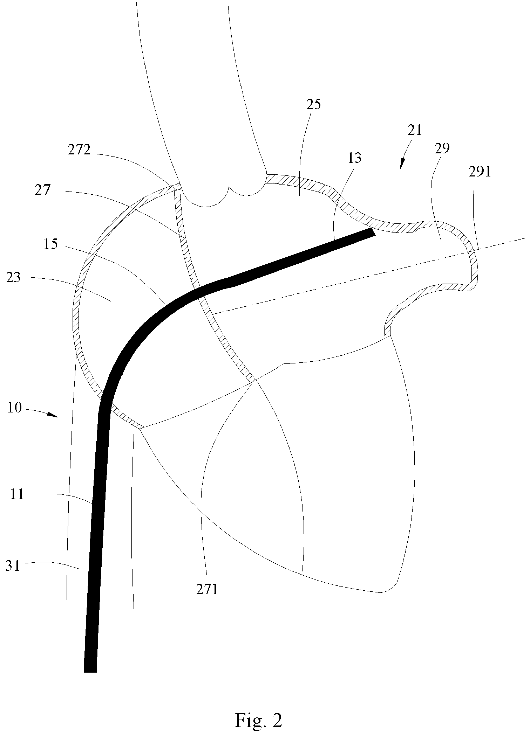

As shown in Fig. 2, a human body includes a heart 21 and an inferior vena cava 31, and the heart 21 includes a right atrium 23, a left atrium 25, an atrial septum 27 and a left atrial appendage 29. The right atrium 23 is communicated with the inferior vena cava 31. The atrial septum 27 is located between the right atrium 23 and the left atrium 29. The atrial septum 27 separates the right atrium 23 from the left atrium 29. The atrial septum 27 has a first end part 271 and a second end part 272. The first end 271 is close to the cardiac apex. The second end part 272 is opposite to the first end part and away from the cardiac apex. The atrial septum has an extending direction from the second end part 272 to the first end part 271. The left atrial appendage 29 is roughly of a barrel-shaped structure and has an opened end and a closed end. The opened end may cause the left atrial appendage 29 to be communicated with the left atrium 25, and the closed end closes the left atrial appendage 29. An acute angle formed by a central axis 291 of the left atrial appendage 29 and the extending direction of the inferior vena cava is approximately 45 degrees.

When the single-bendable pre-shaped delivery sheath 10 is in use, it generally enters the right atrium 23 through the inferior vena cava 31, and then punctures the atrial septum 27 so that the distal section 13 of the single-bendable pre-shaped delivery sheath 10 enters the left atrium 25 and the proximal section 11 is kept within the inferior vena cava 31. The proximal section 11 kept within the inferior vena cava 31 can be considered being coaxial with the inferior vena cava 31. Since the acute angle formed by the central axis 291 of the left atrial appendage 29 and the extending direction of the inferior vena cava 31 is approximately 45 degrees, it is equivalent that the acute angle formed by the central axis 291 of the left atrial appendage 29 and the proximal section 11 is 45 degrees. If a puncture point of the atrial septum 27 is selected correctly, the atrial septum 27 will not change the angle between the proximal section 11 and the distal section 13, that is, the included angle between the proximal section 11 and the punctured distal section 13 is 45 degrees, which is equivalent that the included angle formed between the extending direction of the inferior vena cava 31 and the punctured distal section 13 is 45 degrees. Therefore, the distal section 13 of the single-bendable pre-shaped sheath 10 can extend into the left atrial appendage 29, and the distal section 13 coincides with the central axis 291 of the left atrial appendage 29.

However, in actual operation, an actual puncture point and an ideal puncture point often deviate, and the included angle between the central axis 291 of the left atrial appendage 29 of some patients and the extending direction of the inferior vena cava 31 is less than 45 degrees, which is equivalent that the left atrial appendage 29 takes the closed end as an axis, and the opening of the left atrial appendage 29 deviates a certain angle towards one side of the first end part 271 of the atrial septum 27. During puncturing of the atrial septum 27, when the position for puncturing the atrial septum is not suitable, for example, when the actual puncture point is higher than the ideal puncture point, that is, when the actual puncture point deviates from the ideal puncture point towards a direction away from the cardiac apex in a lengthwise direction of the atrial septum 27, the distal section 13 of the single-bendable pre-shaped delivery sheath 10 may abut against a side wall of the left atrial appendage 29 at the opening of the left atrial appendage 29, resulting in that the distal section 13 of the single-bendable pre-shaped delivery sheath 10 fails in extending into the left atrial appendage, and the distal section 13 of the single-bendable pre-shaped delivery sheath 10 fails in being coaxial with the central axis 291 of the left atrial appendage 29. In this case, when the left atrial appendage occluder is delivered or released, a fixing plate of the left atrial appendage occluder is difficultly fixed on an inner wall of the left atrial appendage 29, and it is easy for the left atrial appendage occluder to fall off and cause device embolism.

During the operation, an operator may move or rotate the proximal end of the single-bendable pre-shaped delivery sheath 10 to change the position of the distal section 13. However, since the puncture point is placed at a higher position, no matter how to move or push and pull the sheath, the distal section 13 cannot be caused to be coaxial with the central axis 29 of the left atrial appendage 29.

### Summary

It is desired to provide a delivery sheath to solve the problem in the prior art that the delivery sheath cannot be coaxial with a left atrial appendage.

A delivery sheath includes a canal body portion. The canal body portion includes an outer canal and a traction structure. The outer canal includes a connecting canal, a canal body, and a distal canal. The canal body is located between the connecting canal and the distal canal. The canal body includes a first flexible canal, a spacer canal, and a second flexible canal. The spacer canal is provided between a distal end of the first flexible canal and a proximal end of the second flexible canal. The hardness of the connecting canal is greater than that of the first and second flexible canals, and the hardness of the spacer canal is greater than that of the first and second flexible canals. The traction structure includes a first traction fiber and a second traction fiber. The first traction fiber is connected to the first flexible canal, and the second traction fiber is connected to the second flexible canal. The first traction fiber and the second traction fiber both extend into the outer canal, and a free end of the first traction fiber and a free end of the second traction fiber extend out of a proximal end of the connecting canal. The first traction fiber can slide with respect to the canal body and can drive the first flexible canal to bend in a first plane. The second traction fiber can slide with respect to the canal body and can drives the second flexible canal to bend in a second plane. The first plane intersects or is parallel to or is coplanar with the second plane.

In one embodiment, a medical device is further provided. The medical device includes the foregoing delivery sheath.

In use, the above-mentioned delivery sheath generally enters the right atrium through the inferior vena cava, and then punctures the atrial septum. After the puncture, the distal canal of the delivery sheath and one portion of the canal body enter the left atrium. The first traction fiber applies a traction force to the distal end of the first flexible canal so that the first flexible canal bends to deform in the first plane and drives the distal canal to move. The second traction fiber applies a traction force to the distal end of the second flexible canal so that the second flexible canal bends to deform in the second plane and drives the distal canal to move. Since the first plane and the second plane intersect each other, the position of the distal canal can be adjusted in two dimensions through the first traction fiber and the second traction fiber, which causes the distal canal to be coaxial with the left atrial appendage. The hardness of the connecting canal is greater than the hardness of the first and second flexible canals to supply an enough axial supporting force to the delivery sheath, so that in a surgical process, the first and second flexible canals bend on a stable structure, which achieves controllable bending and predictable bending forms for the first and second flexible canals and enables the delivery sheath to be quickly coaxial with the left atrial appendage. The hardness of the spacer canal is greater than the hardness of the first and second flexible canals, which can avoid a pivot during bending of the second flexible canal from moving onto the first flexible canal, thus achieving an effect of avoiding the bending of the second flexible canal from affecting the shape of the fist flexible canal and enabling the delivery sheath to be quickly coaxial with the left atrial appendage.

### Brief Description of the Drawings

Fig. 1 is a schematic structural diagram of a single-bendable pre-shaped sheath in the prior art.
Fig. 2 is a state diagram of a single-bendable pre-shaped sheath in use in the prior art.
Fig. 3 is a schematic diagram of a sectional structure of a delivery sheath of one embodiment.
Fig. 4 is a partially sectional view of a delivery sheath of one embodiment.
Fig. 5 is a partially sectional view of a delivery sheath of one embodiment.
Fig. 6 is a three-dimensional view of a first fixed structure of one embodiment.
Fig. 7 is a schematic structural diagram of cooperation between a first traction fiber and a first fixed structure of one embodiment.
Fig. 8 is a schematic structural diagram of a connecting canal of one embodiment.
Fig. 9 is a state diagram of a delivery sheath in use of one embodiment.
Fig. 10 is a cutaway view along A-A in Fig. 9.
Fig. 11 is a schematic structural diagram of a delivery sheath of one embodiment.
Fig. 12 is a schematic structural diagram of a delivery sheath of one embodiment.
Fig. 13 is a schematic diagram of a sectional structure of a delivery sheath of one embodiment.
Fig. 14 is a schematic diagram of a sectional structure of a delivery sheath of one embodiment.
Fig. 15 is a schematic structural diagram of a supporting member of one embodiment.
Fig. 16 is a schematic structural diagram of a supporting member of one embodiment.
Fig. 17 is a schematic structural diagram of a supporting member of one embodiment.
Fig. 18 is a schematic structural diagram of a supporting member of one embodiment.
Fig. 19 is a schematic structural diagram of a supporting member of one embodiment.

### Detailed Description of the Embodiments

In order to make the foregoing objectives, features and advantages of the embodiments clearer, the implementation modes of the embodiments are described in detail with reference to the accompanying drawings. Many specific details are described in the following descriptions to facilitate full understanding of the embodiments. However, the embodiments can be implemented in a variety of other ways than those described herein, and those skilled in the art can make similar improvements without departing from the connotation of the embodiments. Therefore, the embodiments are not limited by specific implementations below.

It should be noted that when an element is referred to as being "fixed" or "arranged" to another element, it can be directly on the other element or an intermediate element may also exist. When an element is referred to as being "connected" to another element, it can be directly connected to the other element or an intermediate element may exist at the same time. The terms "perpendicular", "horizontal", "left", "right" and similar expressions used herein are for illustrative purposes only, and are not meant to be the only implementation modes.

Unless otherwise defined, all technical and scientific terms used herein are the same as meanings of general understandings of those skilled in the art of the embodiments. The terms used in the description herein are merely to describe the implementation modes, not intended as limiting the embodiments. The term "and/or" used herein includes any and all combinations of one or more related listed items.

In order to describe the structure of the embodiments more clearly, "distal end" and "proximal end" are used as orientation words, where "distal end" means an end away from an operator during the operation, and "proximal end" means an end close to the operator during the operation.

### First embodiment

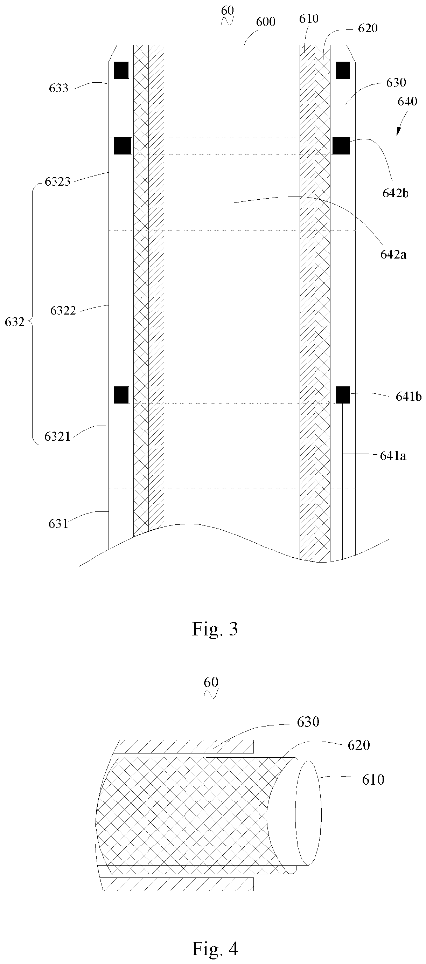

Referring to Fig. 3, a delivery sheath 60 provided in this embodiment is a bendable sheath. The delivery sheath 60 includes a canal body portion 600. The canal body portion 600 is linear in a natural state.

The canal body portion 600 includes an inner canal 610, an intermediate layer 620, an outer canal 630, and a traction structure 640.

The inner canal 610 is made of a high-molecular material, so it has high lubricity and low friction. An inner surface of the inner canal is smooth, which can ensure that an implanted device (such as a left atrial appendage occluder, not shown) passes through the inner surface smoothly. The high-molecular material may be polytetrafluoroethylene (PTFE).

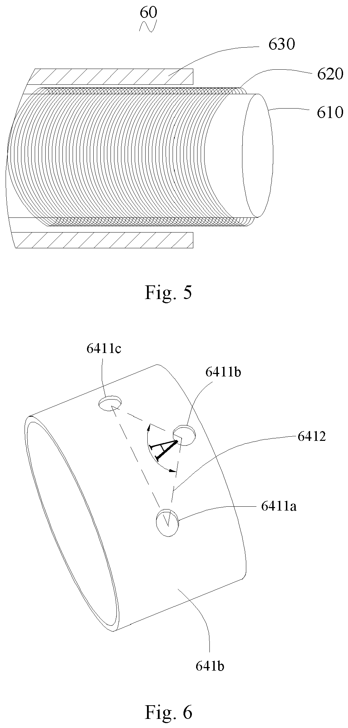

The intermediate layer 620 is a metal layer pasted on an outer surface of the inner canal 610. Referring to Fig. 4, the intermediate layer 620 may be a braided tube braided by a metal wire. Referring to Fig. 5, the intermediate layer 620 may also be a metal spring tube. The braided tube is formed by braiding the metal wire with a braiding machine. The metal spring tube is wound by a spring machine for winding springs.

The outer canal 630 is made of high-molecular materials with different hardness (or different elasticity), such as Pebax.

In a process of making the delivery sheath 60, the intermediate layer 620 is tightly bundled and attached to an outer surface of the inner canal 610 and is then sleeved with the outer canal 630. Hot-melt molding is performed. After the hot melting, the outer surface of the inner canal 610 is connected to the inner surface of the outer canal 630, so that the inner canal 610, the intermediate layer 620, and the outer canal 630 form an integrated structure.

Referring to Fig. 3 again, the outer canal 630 includes a connecting canal 631, a canal body 632 and a distal canal 633. The connecting canal 631 is located at a proximal end of the canal body 632. The distal canal 633 is located at a distal end of the canal body 632. The canal body 632 is located between the connecting canal 631 and the distal canal 633. One end of the canal body 632 is connected to the connecting canal 631, and the other end of the canal body 632 is connected to the distal canal 633.

The canal body 632 includes a first flexible canal 6321, a spacer canal 6322, and a second flexible canal 6323. A proximal end of the first flexible canal 6321 is connected to the connecting canal 631. The spacer canal 6322 is a straight canal in its natural state, and the spacer canal 6322 is provided between a distal end of the first flexible canal 6321 and a proximal end of the second flexible canal 6323. A distal end of the second flexible canal 6323 is connected to the proximal end of the distal canal 633. The first flexible canal 6321 and the second flexible canal 6323 are of bendable tubular structures, that is, the first flexible canal 6321 and the second flexible canal 6323 can bend to deform under the action of a traction force. The hardness of the connecting canal 631 is greater than that of the first flexible canal 6321 and the second flexible canal 6323, and the hardness of the spacer canal 6322 is greater than that of the first flexible canal 6321 and the second flexible canal 6323. The hardness of the distal canal 633 is greater than that of the first flexible canal 6321 and the second flexible canal 6323. When the first flexible canal 6321 and the second flexible canal 6323 bend to deform under the action of the traction force, the connecting canal 631, the spacer canal 6322, and the distal canal 633 do not bend to deform.

The traction structure 640 includes a first traction fiber 641a, a second traction fiber 642a, a first fixed structure 641b, and a second fixed structure 642b,

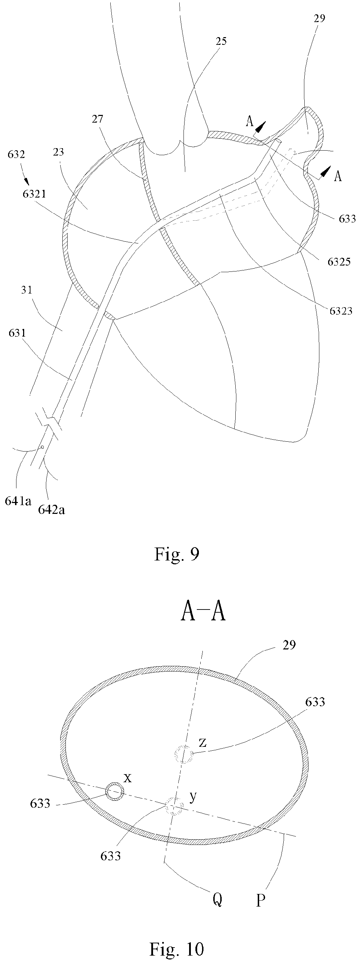

The first traction fiber 641a is connected to the distal end of the first flexible canal 6321. The second traction fiber 642a is connected to the distal end of the second flexible canal 6323. Both the first traction fiber 641a and the second traction fiber 642a linearly extend in the outer canal 630. A free end of the first traction fiber 641a and a free end of the second traction fiber 642a extend out of the proximal end of the connecting canal 631. The first traction fiber 641a is slidable relative to the canal body 632 and can drive the first flexible canal 6321 to bend in the first plane P (shown in Fig. 10) towards one side under a force. The second traction fiber 642a is slidable relative to the canal body 632 and can drive the second flexible canal 6323 to bend in the second plane Q (shown in Fig. 10) towards one side under a force. The first plane P intersects the second plane Q.

Both the first fixed structure 641b and the second fixed structure 642b are fixed in the canal body 632, and both the first fixed structure 641b and the second fixed structure 642b are rings. In an embodiment, the first fixed structure 641b is arranged at the distal end of the first flexible canal 6321. The distal end of the first fixed structure 641b is flush with the distal end of the first flexible canal 6321. The first fixed structure 641b is embedded in the first flexible canal 6321. The second fixed structure 642b is arranged at the distal end of the second flexible canal 6323. The distal end of the second fixed structure 642b is flush with the distal end of the second flexible canal 6323. The second fixed structure 642b is embedded in the second flexible canal 6323.

Three connecting holes are formed in side walls of the first fixed structure 641b and the second fixed structure 642b. Since the distributions of the connecting holes on the first fixed structure 641b and the second fixed structure 642b are the same. Only the connecting holes on the first fixed structure 641b are described here. Referring to Fig. 6, the side wall of the first fixed structure 641b is provided with a first connecting hole 6411a, a second connecting hole 6411b, and a third connecting hole 6411c. Along a circumferential direction of the first fixed structure 641b, the second connecting hole 6411b is located between the first connecting hole 6411a and the third connecting hole 6411c. Connecting lines of hole centers of the first connecting hole 6411a, the second connecting hole 6411b, and the third connecting hole 6411c may form a triangle 6412. An inner angle A formed by the triangle 6412 at the hole center of the second connecting hole 6411b is an obtuse angle. The first traction fiber 641a passes through the first connecting hole 6411a, the second connecting hole 6411b, and the third connecting hole 6411c in sequence to be connected with the first fixed structure 641b. The second traction fiber 642a passes through the three connecting holes 6411 of the second fixed structure 642b in sequence to be connected with the second fixed structure 642b.

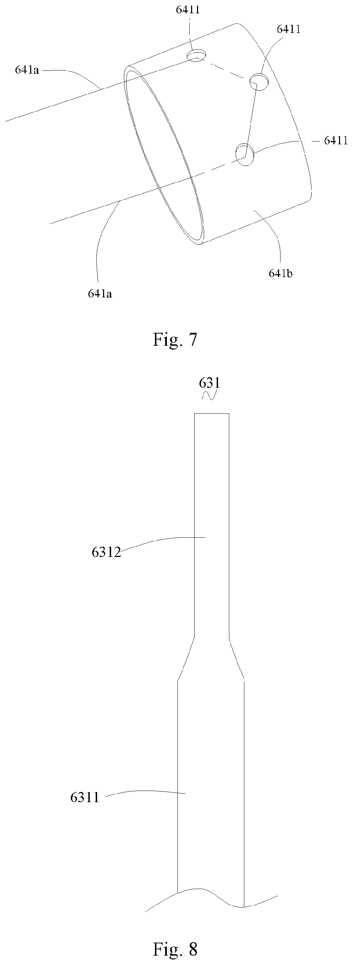

Referring to Fig. 7, the holes center of the first connecting hole 6411a and the third connecting hole 6411c are located on the same circumferential line of the first fixed structure 641b. The first traction fiber 641a passes through the first connecting hole 6411a, the second connecting hole 6411b, and the third connecting hole 6411c in sequence to be connected with the first fixed structure 641b, so that included angles formed by the first traction fiber 641a at the first connecting hole 6411a, the second connecting hole 6411b, and the third connecting hole 6411c of the first fixed structure 641b are all obtuse angles, thereby avoiding stress concentration during use of the first traction fiber 641a and prolonging the service life.

It can be understood that the structure of the second fixed structure 642b is the same as that of the first fixed structure 641b, and a connection way for the second traction fiber 642a and the second fixed structure 642b is the same as that for the first traction fiber 641a and the first fixed structure 641b, which can also avoid stress concentration during use of the second traction fiber 642a.

In other embodiments, the traction structure 640 only includes the above-mentioned first fixed structure 641b and the first traction fiber 641a connected thereto, or, the traction structure 640 only includes the above-mentioned second fixed structure 642b and the second traction fiber 642a connected thereto.

The hardness of the first flexible canal 6321 ranges from 10D to 40D, which can ensure that the first flexible canal 6321 is bendable in the first plane P. The hardness of the second flexible canal 6323 ranges from 10D to 40D, which can ensure that the second flexible canal 6323 is bendable in the second plane Q.

The hardness of the connecting canal 631 ranges from 55D to 72D, that is, the hardness of the connecting canal 631 is greater than that of the first flexible canal 6321 and the second flexible canal 6323. In this way, when the delivery sheath 60 meets a requirement for adapting to the shape of a blood vessel, the connecting canal 631 provides an enough axial supporting force for the delivery sheath 60, so that during the operation, the first flexible canal 6321 and the second flexible canal 6323 bend on a stable structure, which achieves controllable bending and predictable bending forms for the first flexible canal 6321 and the second flexible canal 6323 and enables the delivery sheath 60 to be quickly coaxial with the left atrial appendage. The hardness of the spacer canal 6322 ranges from 55D to 72D, that is, the hardness of the spacer canal 6322 is greater than that of the first flexible canal 6321 and the second flexible canal 6323, which can avoid a pivot during bending of the second flexible canal 6323 from moving onto the first flexible canal 6321, thus achieving an effect of avoiding the bending of the second flexible canal 6323 from affecting the shape of the fist flexible canal 6321 and enabling the delivery sheath 60 to be quickly coaxial with the left atrial appendage. The hardness of the distal canal 633 ranges from 55D to 72D, which can ensure the axial strength of the distal canal 633, so that the distal canal 633 is always kept in a straight shape when the implanted device is released or withdrawn, ensuring that the implanted device can be released and withdrawn smoothly. In an embodiment, the connecting canal 631, the spacer canal 6322, and the distal canal 633 have the same hardness that is 72D, which can provide an enough axial supporting force under the condition that the delivery sheath 60 meets the requirement for adapting to the shape of a blood vessel.

The hardness of the first flexible canal 6321 is greater than or equal to that of the second flexible canal 6323, which can also prevent the second flexible canal 6323 from affecting the shape of the first flexible canal 6321 when the second flexible canal 6323 bends, so that the phenomenon that the distal canal 633 cannot be coaxial with the left atrial appendage due to the influence of the second flexible canal 6323 on the shape of the first flexible canal 6321 can be avoided.

The first traction fiber 641a is made of a high-strength material, such as carbon fiber yarn or nickel-titanium yarn. The first traction fiber 641a is a multi-strand fiber. Compared with a single fiber, the multi-strand fiber has less fatigue stress. When it is connected to the first fixed structure 641b, breakage caused by concentration of the fatigue stress is unlikely to occur. Even if a certain fiber in the first traction fiber 641a is broken, other fibers may also adjust the angle of the sheath. The service life of the first traction fiber 641a can be prolonged. Furthermore, when a certain fiber of the first traction fiber 641a is broken, an abnormal or unexpected sound (for example, "bang") will be made, which can remind the operator, for example that "a certain fiber of the first traction fiber 641a has been damaged. Be careful. End this operation as soon as possible."

The structure and material of the second traction fiber 642a are the same as those of the first traction fiber 641a, and both of the traction fibers are multi-strand fibers.

Referring to Fig. 8, the connecting canal 631 includes a proximal portion 6311 and a distal portion 6312. The distal portion 6312 is connected to the first flexible canal 6321 of the canal body 632. The delivery sheath 60 has a first wall thickness within a length range of the proximal portion 6311, and the delivery sheath 60 has a second wall thickness within a length range of the distal portion 6312. The first wall thickness is greater than the second wall thickness, thus enhancing the strength of the delivery sheath 60 in the length range of the proximal portion 6311. When the delivery sheath 60 bends, the traction structure 640 can be avoided from driving the canal body to bend to deform within the length range of the proximal portion 6311 of the delivery sheath 60 (that is, the "hunchback" deformation of the corresponding delivery sheath 60 within the length range of the proximal portion 6311 can be avoided). This in turn avoids compression of blood vessels and internal organs caused by the "hunchback" of the delivery sheath 60.

In an embodiment, the delivery sheath 60 is provided with a layer of high-hardness high-molecular material outside its outer canal 630, and the hardness of the high-molecular material with higher hardness ranges from 63D to 72D. The high-molecular material with higher hardness is arranged in the length range corresponding to the proximal portion 6311, which can improve the wall thickness and strength of the delivery sheath 60 in the length range of the proximal portion 6311. Alternatively, the thickness of the intermediate layer 620 within the length range of the proximal portion 6311 is increased to enhance the wall thickness and strength of the delivery sheath 60 within the length range of the proximal portion 6311.

An inner diameter of the delivery sheath 60 in each section is the same. The portion of the delivery sheath 60 at the first wall thickness has a first outer diameter, and the portion of the delivery sheath 60 at the second wall thickness has a second outer diameter. The first outer diameter is greater than the second outer diameter. The smooth transition from the first outer diameter of the delivery sheath 60 to the second outer diameter can reduce injury of the delivery sheath 60 to the blood vessels and internal organs of a patient.

It may be appreciated that, in other embodiments, the inner diameter and outer diameter of the delivery sheath 60 in each section are the same, and the wall thickness of the delivery sheath 60 in each section is the same.

Referring to Fig. 9 and Fig. 10 together, when the delivery sheath 60 is used, the delivery sheath 60 enters the right atrium 23 from the inferior vena cava 31, and then punctures the atrial septum 27. After the puncture, the distal canal 633 of the delivery sheath 60 and one portion of the canal body 632 enter the left atrium 25. The first traction fiber 641a applies a traction force to the distal end of the first flexible canal 6321 so that the first flexible canal 6321 bends to deform in the first plane P and the distal canal 633 can move from position X to position Y. The second traction fiber 642a applies a traction force to the distal end of the second flexible canal 6323 so that the second flexible canal 6323 bends to deform in the second plane Q and the distal canal 633 can move from position Y to position Z. So far, the distal canal 633 is coaxial with the left atrial appendage 29. Since the first plane P intersect the second plane Q, the position of the distal canal 633 can be adjusted in two dimensions by the first traction fiber 641a and the second traction fiber 642a. The delivery sheath 60 of this embodiment can be applicable to hearts in all anatomical shapes. The position of the distal canal 633 of the delivery sheath 60 can be adjusted in two dimensions to ensure that the distal canal 633 is coaxial with the left atrial appendage 29, thereby improving the convenience of the operation of the delivery sheath 60. At the same time, the operation time can also be saved, and injury to the patient can be lowered.

The spacer canal 6322 is arranged between the first flexible canal 6321 and the second flexible canal 6323, and the hardness of the spacer canal 6322 is greater than that of the first flexible canal 6321 and the second flexible canal 6323. When the second traction fiber 642a is operated to bend the second flexible canal 6323, a pivot when the second flexible canal 6323 bends can be locked at an end part of the second flexible canal 6323 that abuts against the spacer canal 6322 (that is, the proximal end of the second flexible canal 6323), so as to avoid the bending of the second flexible canal 6323 from affecting the shape of the first flexible canal 6321, thus avoiding the phenomenon that the distal canal 633 cannot be coaxial with the left atrial appendage due to the influence of the second flexible canal 6323 on the shape of the first flexible canal 6321.

For example, the first plane P is perpendicular to the second plane Q. The first flexible canal 6321 bends in the first plane P, and the second flexible canal 6323 bends in the second plane Q, which can further improve the bending accuracy of the delivery sheath 60, reduce the operation time and improve the convenience of operation.

In other embodiments, the first plane P is coplanar in parallel with the second plane Q, or the first plane P is parallel to the second plane Q. When the first plane P is coplanar in parallel with the second plane Q, it can be avoided that the delivery sheath 60 cannot be coaxial with the left atrial appendage due to the fact that a puncture point deviates backward or forward. It can be understood that the puncture point deviating backward means that in a plane where the atrial septum is located, along the extending direction of the atrial septum, the puncture point deviates towards the human back. The forward deviation direction of the puncture point is opposite to the backward deviation direction of the puncture point. When the first plane P is parallel to the second plane Q, the phenomenon that the delivery sheath 60 cannot be coaxial with the left atrial appendage due to the forward or backward deviation of the puncture point can be avoided, and the phenomenon that the delivery sheath 60 cannot be coaxial with the left atrial appendage due to a higher or lower puncture point can also be avoided. It should be understood that the lower puncture point means that in the plane where the atrial septum is located, along a direction from the extending direction of the atrial septum towards the cardiac apex. The direction of the higher puncture point is opposite to the direction of the lower puncture point.

### Second embodiment

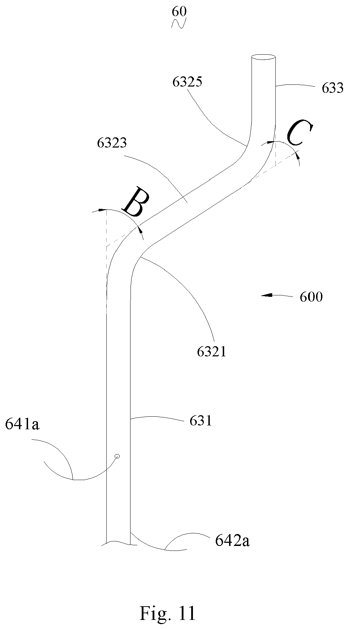

Referring to Fig. 11, a difference between the second embodiment and the first embodiment is that the canal body portion 600 is bent in a natural state. In the embodiment, the first flexible canal 6321 is pre-shaped into a bent canal structure in the first plane P, and the extending direction of a proximal end part of the first flexible canal 6321 and the extending direction of a distal end part of the first flexible canal 6321 form an included angle B which ranges from 45 degrees to 90 degrees.

When the second flexible canal 6323 is in a natural state, the second flexible canal 6323 is pre-shaped in the second plane Q into a bent canal structure, and the extending direction of the second flexible canal 6323 at a proximal end part and the extending direction of the second flexible canal 6323 at a distal end part form an included angle C which ranges from 30 degrees to 60 degrees.

On the basis of the pre-shaping of the first flexible canal 6321 and the second flexible canal 6323, only the shape of the delivery sheath 60 needs to be finely adjusted, so that the distal canal 633 of the delivery sheath 60 is coaxial with the left atrial appendage.

In other embodiments, one of the first flexible canal 6321 and the second flexible canal 6323 is bent in its natural state, that is, the extending direction of the proximal end part of the first flexible canal 6321 and the extending direction of the distal end part of the first flexible canal 6321 forms an included angle B which ranges from 45 degrees to 90 degrees. Alternatively, the extending direction of the proximal end part of the second flexible canal 6323 and the extending direction of the distal end part of the second flexible canal 6323 form an included angle C which ranges from 30 degrees to 60 degrees.

### Third embodiment

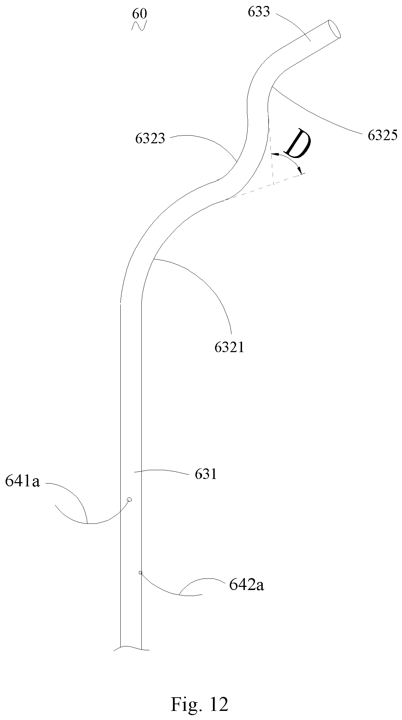

Referring to Fig. 12, a difference between this embodiment and the second embodiment is that the spacer canal 6322 is bent in the second plane Q in its natural state. For example, the spacer canal 6322 can be in a bent shape in the second plane Q by pre-shaping. The spacer canal 6322 bends in an opposite direction to the second flexible canal 6323, so that the delivery sheath 60 is "S"-shaped in its natural state. The extending direction of the proximal end part of the spacer canal 6322 and the extending direction of the distal end part of the spacer canal 6322 form an included angle D which ranges from 30 degrees to 60 degrees.

### Fourth embodiment

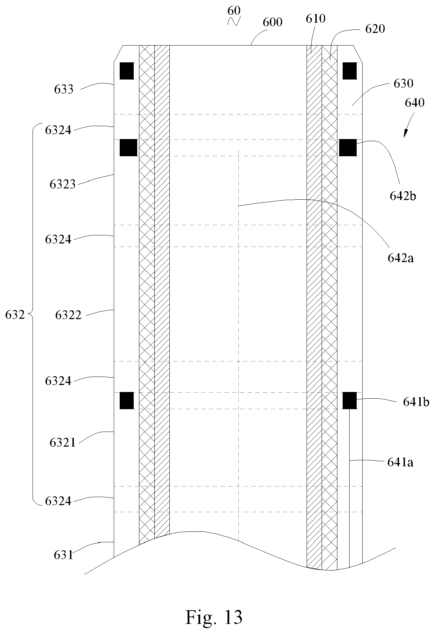

Referring to Fig. 13, a difference between this embodiment and the first embodiment is that the canal body 632 further includes a transition canal 6324. The number of the transition canal 6324 may be one or plural. The hardness of the transition canal 6324 is less than that of the connecting canal 631, the spacer canal 6322, and the distal canal 633. The hardness of the transition canal 6324 is greater than that of the first flexible canal 6321 and the second flexible canal 6323. The hardness of the transition canal 6324 ranges from 40D to 55D.

In this embodiment, the number of the transition canals 6324 is four. In the embodiment, one transition canal 6324 is arranged between the distal canal 633 and the second flexible canal 6323. When the implanted device is withdrawn, it can be avoided that the delivery sheath 60 bends at a junction between the second flexible canal 6323 and the distal canal 633 due to a large difference in the hardness of the second flexible canal 6323 and the distal canal 633. One transition canal 6324 is arranged between the connecting canal 631 and the first flexible canal 6321. When the implanted device is withdrawn, it can be avoided that the delivery sheath 60 bends at a junction between the first flexible canal 6321 and the connecting canal 631 due to a large difference in the hardness of the first flexible canal 6321 and the connecting canal 631. One transition canal 6324 is arranged between the spacer canal 6322 and the first flexible canal 6321. When the implanted device is withdrawn, it can be avoided that the delivery sheath 60 bends at a junction between the first flexible canal 6321 and the spacer canal 6322 due to a large difference in the hardness of the first flexible canal 6321 and the spacer canal 6322. One transition canal 6324 is arranged between the spacer canal 6322 and the second flexible canal 6323. When the implanted device is withdrawn, it can be avoided that the delivery sheath 60 bends at a junction between the second flexible canal 6323 and the spacer canal 6322 due to a large difference in the hardness of the second flexible canal 6323 and the spacer canal 6322. This ensures that he implanted device can be safely withdrawn through the delivery sheath 60.

It may be appreciated that, in other embodiments, the number and arrangement position of the transition canals 6324 can also be selected according to actual needs. For example, the number of transition canal 6324 is one. The transition canal 6324 may be arranged between the spacer canal 6322 and the second flexible canal 6323. Alternatively, the transition canal 6324 is arranged between the spacer canal 6322 and the first flexible canal 6321. Alternatively, the transition canal 6324 is arranged between the connecting canal 631 and the first flexible canal 6321. Alternatively, a transition canal 6324 is arranged between the distal canal 633 and the second flexible canal 6323.

### Fifth embodiment

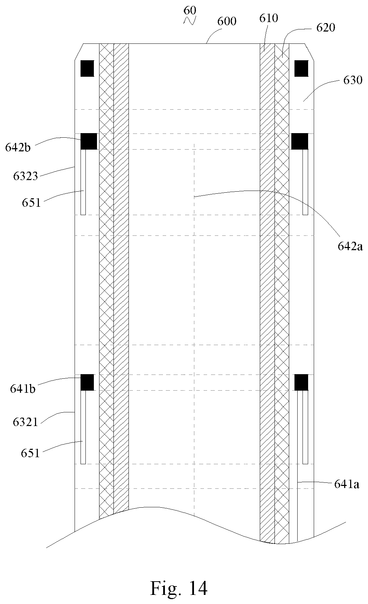

Referring to Fig. 14, a difference between the fifth embodiment and the fourth embodiment is that the delivery sheath 60 in this embodiment further includes a supporting member 651, and the number of the supporting member 651 is at least one. The supporting member 651 is arranged on an outer side of an outer surface of the intermediate layer 620, and the supporting member 651 is positioned on an inner side of an outer surface of the outer canal 630.

In this embodiment, the number of the supporting members 651 is plural. The supporting members 651 are arranged in sections where the first flexible canal 6321 and the second flexible canal 6323 are located. In the section where the first flexible canal 6321 is located, a distal end of the supporting member 651 is fixedly connected to the first fixed structure 641b, so that the distal end of the supporting member 651 and the first fixed structure 641b jointly form a ring end surface, which can avoid the distal end of the supporting member 651 from being too sharp. In the process that the first flexible canal 6321 restores from the bent shape to its natural state, the outer canal 630 will not be punctured even if the supporting member 651 slides relatively in the outer canal 630, thereby avoiding organ tissues from being punctured. In the section where the second flexible canal 6323 is located, the distal end of the supporting member 651 is fixedly connected to the second fixed structure 642b, so that the distal end of the supporting member 651 and the first fixed structure 642b jointly form a ring end surface, which can avoid the distal end of the supporting member 651 from being too sharp. In the process that the second flexible canal 6323 restores from the bent shape to its natural state, the outer canal 630 will not be punctured even if the supporting member 651 slides relatively in the outer canal 630, thereby avoiding organ tissues from being punctured.

The supporting member 651 may be a super-elastic wire rod. A wire diameter of the supporting member 651 is less than the wall thickness of the outer canal 630, so that the supporting member 651 is arranged between the outer canal 630 and the intermediate layer 620.

The supporting member 651 may bend in the first plane P with the first flexible canal 6321 under the traction action of the first traction fiber 641a. The supporting member 651 bends in the second plane Q with the second flexible canal 6323 under the traction action of the second traction fiber 642a. Since the supporting member 651 has superelasticity, the supporting member 651 will not hinder the bending deformation of the first flexible canal 6321 and the second flexible canal 6323, and the supporting member 651 can increase axial supporting forces of the first flexible canal 6321 and the second flexible canal 6323, so that the axial supporting force of the delivery sheath 60 in the first flexible canal 6321 and the second flexible canal 6323 can be increased, and the delivery sheath 60 increases its axial supporting force on the basis of the bending performance. The delivery sheath 60 has a stable shape, so that the implanted device can be delivered and withdrawn smoothly.

After the traction force of the first traction fiber 641a and the second traction fiber 642a is removed, in the process that the first flexible canal 6321 restores to the shape in its natural state by the superelastic action of the supporting member 651, and in the process that the second flexible canal 6323 restores to the shape in its natural state by the superelastic action of the supporting member 651, the superelasticity of the supporting member 651 makes up for the elastic hysteresis effect of the outer canal 630.

In other embodiments, the number of supporting members 651 can also be 3, 4 or more, and the delivery sheath 60 is provided with a plurality of supporting members 651 along its circumference in the first flexible canal 6321, which can further improve the axial supporting force of the bent portion of the delivery canal 60. The delivery sheath 60 is provided with a plurality of supporting members 651 along its circumferential direction in the second flexible canal 6323, which can further improve the axial supporting force of the second flexible canal 6323.

In other embodiments, the number of supporting member 651 may also be one, and the supporting member 651 may be arranged in the section where one of the second flexible canal 6323 and the first flexible canal 6321 is located.

A supporting member 661 in other embodiments may also be a sheet material. The wall thickness of the supporting member 661 is less than the wall thickness of the outer canal 630 so that the supporting member 661 is more easily embedded in the outer canal 630.

### Sixth embodiment

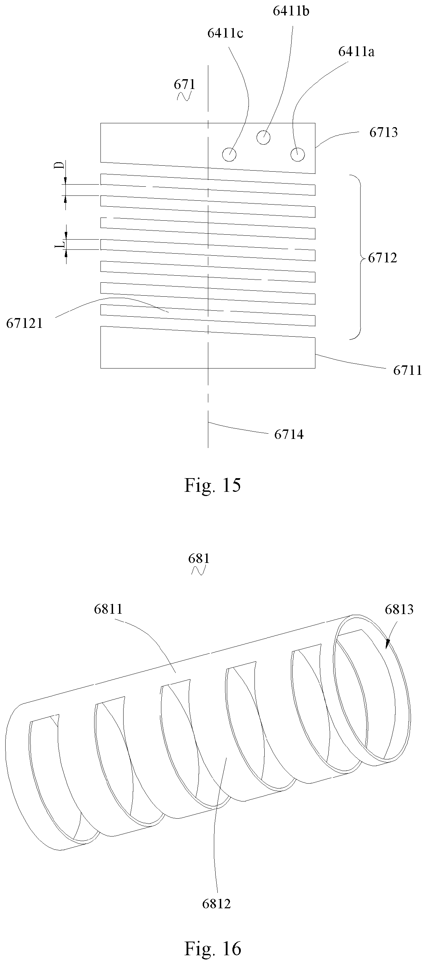

Referring to Fig. 15, a difference between the sixth embodiment and the fifth embodiment is that the supporting member 671 in this embodiment is a spiral body. The supporting member 671 is sleeved on the outer side of the outer surface of the intermediate layer 620, and the supporting member 671 is located on the inner side of the outer surface of the outer canal 630. The supporting member 671 is made of a super-elastic material. The supporting member 671 includes a proximal section 6711, a spiral extension section 6712, and a distal section 6713. The spiral extension section 6712 is located between the proximal section 6711 and distal section 6713.

The proximal section 6711 of the supporting member 671 is a closed ring, and a distal end of the proximal section 6711 is connected to the spiral extension section 6712.

The distal section 6713 of the supporting member 671 is a closed ring. The first connecting hole 6411a, the second connecting hole 6411b, and the third connecting hole 6411c may not be provided on the fixed structure, but are provided on a side wall of the distal section 6713 of the supporting member 671, which can avoid stress concentration in the use process of the first traction fiber 641a and the second traction fiber 642a and can also save the material of one fixed structure and reduce the cost.

The spiral extension section 6712 has an extension 67121. The extension 67121 can be formed by performing laser cutting on a pipe in the spiral extension section 6712. The extension body 67121 extends spirally in the spiral extension section 6712.

A side wall of the supporting member 671 is cut along a direction parallel to a central axis 6714 of the supporting member 671, and the cut supporting member 671 is spread along a radial direction of the supporting member 671. An included angle E formed by the extension body 67121 and the central axis 6714 of the supporting member 671 ranges from 0 to 90 degrees, so that an enough axial supporting force can be provided when it is ensured that the supporting member 671 is bendable. In an example, the included angle E formed by the extension body 67121 and the central axis 6714 of the supporting member 671 ranges from 60 degrees to 90 degrees. In the bending process of the delivery sheath, the fracture resistance of the delivery sheath 60 is improved, and the axial supporting force of the delivery sheath 60 is improved at the same time. An axial thickness L of the extension body 67121 is 0.05 mm-3.0 mm. In the bending process of the delivery sheath, the fracture resistance of the delivery sheath is improved, and the axial supporting force of the delivery sheath is improved at the same time. In a further example, an axial thickness L of the extension body 67121 is 0.1 mm-0.5 mm. A gap D between two adjacent extension bodies 67121 is 0.05 mm-3.0 mm. Additionally, in the bending process of the delivery sheath, by the gap D between two adjacent extension bodies 67121, the fracture resistance of the delivery sheath is improved, and the axial supporting force of the delivery sheath is improved at the same time. It can be understood that if the included angle E formed by the extension body 67121 and the central axis of the supporting member 671 is smaller, the gap D between two adjacent extension bodies 67121 is larger, and the supporting member 671 has higher bendability. If the included angle E is larger, the gap D between two adjacent extension bodies 67121 is smaller, and the axial supporting force of the supporting member 671 is higher.

### Seventh embodiment

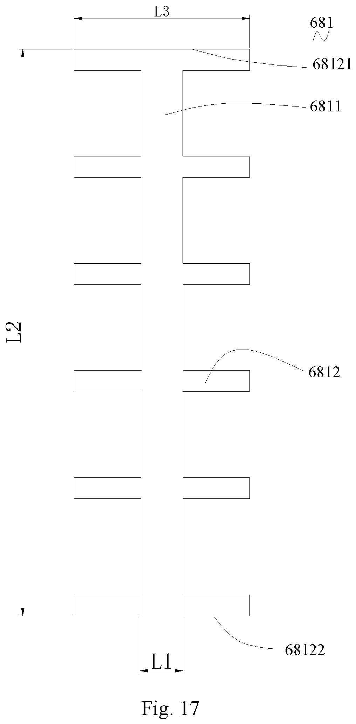

Referring to Fig. 16 and Fig. 17, a difference between the seventh embodiment and the sixth embodiment is that the supporting member 681 in this embodiment is a keel structure.

In this embodiment, the sections where the first flexible canal 6321 and the second flexible canal 6323 are located are provided with supporting members 681. The supporting members 681 include one keel 6811 and a plurality of skeletons 6812. The plurality of skeletons 6812 are arranged along an axial direction (a lengthwise direction) of the keel 6811 at intervals. Two ends of the plurality of skeletons 6812 are respectively connected with two sides of the keel 6811. The keel 6811 and the plurality of skeletons 6812 together form a ring-shaped accommodating space 6813. The intermediate layer 620 (that is, a canal wall of the delivery sheath 60) is accommodated in the accommodating space 6813 so that the supporting member 681 is sleeved outside the intermediate layer 620 (that is, the supporting member 681 is arranged in the delivery sheath 60). When the distal end of the supporting member 681 is subjected to a traction force, all the skeletons 6812 are spaced apart from each other, so that there is a space for deformation between the skeletons 6812, which can reduce the bending resistance of the supporting member 681 to ensure that the supporting member 681 does not hinder the bending of the first flexible canal 6321 and the second flexible canal 6323. At the same time, all the skeletons 6812 are spaced apart from one another, so that the high-molecular material of the outer canal 630 can be embedded, which can avoid the supporting member 681 from axially sliding with respect to the outer canal 630, thus avoiding the proximal end of the supporting member 681 from piercing the high-molecular material of the outer canal 630. In addition, since the keel 6811 can provide an axial supporting force, the supporting member 681 can improve the axial supporting force of the first flexible canal 6321 and the second flexible canal 6323 and avoid the delivery sheath 60 from bending.

Of course, in other embodiments, the section where the supporting member 681 is located can also be selected according to actual needs. For example, the supporting member 681 is provided only in the section where the first flexible canal 6321 is located. Alternatively, the supporting member 681 is provided only in the section where the second flexible canal 6323 is located.

The most distal skeleton 6812 of the plurality of skeletons 6812 has a distal end surface 68121. A distal end part of the keel 6811 is flush with the distal end surface 68121, or the distal end part of the keel 6811 is located on a proximal side of the distal end surface 68121. It can be avoided that the distal end of the keel 6811 pierces the outer canal 630 when the delivery sheath 60 bends.

The most proximal skeleton 6812 of the plurality of skeletons 6812 has a proximal end surface 68122. A proximal end part of the keel 6811 is flush with the proximal end surface 68122, or the proximal end part of the keel 6811 is located on a distal side of the proximal end surface 68122. It can be avoided that the distal end of the keel 6811 pierces the outer canal 630 when the delivery sheath 60 bends.

The distal end of the supporting member 681 is provided with the above-mentioned first connecting hole 6411a, second connecting hole 6411b and third connecting hole 6411c, which can avoid stress concentration in the use process of the first traction fiber 641a and the second traction fiber 642a and can also save the material of one fixed structure and reduce the cost. In an example, the first connecting hole 6411a, the second connecting hole 6411b, and the third connecting hole 6411c may be provided at the distal end of the keel 6811. Alternatively, the first connecting hole 6411a, the second connecting hole 6411b, and the third connecting hole 6411c may be provided on the skeleton 6812 at the distal end of the supporting member 681.

When the supporting member 681 is mounted in the first flexible canal 6321, a length L2 of the supporting member 681 is equal to that of the first flexible canal 6321. When the supporting member 681 is mounted in the second flexible canal 6323, the length L2 of the supporting member 681 is equal to that of the second flexible canal 6323. A width L1 of the keel 6811 is less than half of a circumference L3 of either skeleton 6812, i.e. L1VL3/2. In this way, the hardness of the keel 6811 is moderate, so that while improving the axial supporting force of the delivery sheath 60, it is ensured that the delivery sheath 60 can bend to deform under the driving of the first traction fiber 641a and the second traction fiber 642a. The supporting member 681 may be formed by performing laser cutting on a super-elastic nickel-titanium alloy pipe.

### Eighth embodiment

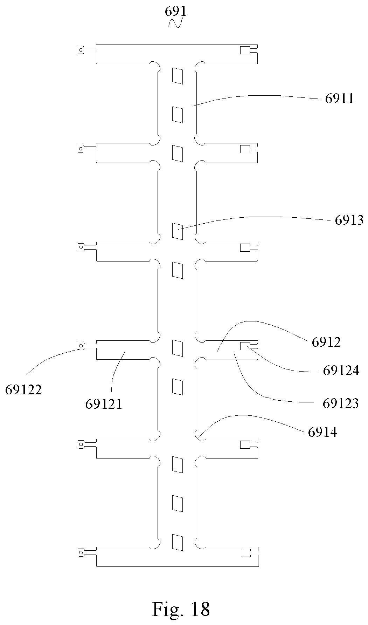

Referring to Fig. 18, a difference between the eighth embodiment and the seventh embodiment is that the keel 691 is also provided with a hollow structure 6913. Elastic nylon of the outer canal 630 can enter the hollow structure 6913, which enlarges a contact area between the keel 691 and the outer canal 630 and can improve the bonding strength of the supporting member 691 and the elastic nylon of the outer canal 630, improve the fatigue life of the supporting member 691 and prolong the service cycle. The hollow structure 6913 can be rectangular, round, elliptical, square, triangular, diamond-shaped, and the like. In an embodiment, the hollow structure 6913 is diamond-shaped. The hollow structure 6913 is diamond-shaped, which improves the bending deformation performance of the supporting member 691, thereby reducing the obstruction effect of the supporting member 691 on the bending deformation of the first flexible canal 6321 and the second flexible canal 6323. A part of the keel 6911 that is connected with the skeleton 6912 is provided with a groove 6914, so that there is a deformation space for the material of the keel 6911 during bending deformation to improve the compliance of the supporting member 691 during bending.

The skeleton 6912 includes a first connecting part 69121 and a second connecting part 69123 that are relatively disposed on both sides of the keel 6911. The first connecting part 69121 and the second connecting part 69123 are symmetrical about the axis of the keel 6911. A free end of the first connecting part 69121 is provided with a buckle 69122. In an embodiment, the buckle 69122 is T-shaped. A free end of the second connecting part 69123 is provided with a slot 69124. The slot 69124 is elastic. A slot width of the slot 69124 can increase within a certain range under the action of an external force on the slot. That is, under the action of the external force, the size of the slot 69124 along the axis of the keel 6912 can increase to a certain extent. The shape of the slot 69124 is matched with the buckle 69122, so that the slot 69124 can accommodate the buckle 69122, and the slot 69124 and the buckle 69122 are in buckling fit to form an accommodating space 6813. The buckle 69122 is inserted into the slot 69124 so that the first connecting part 69121 and the second connecting part 69123 are clamped. Due to the elasticity of the slot 69124, the slot width of the slot 69124 can increase within a certain range under the action of the external force, and the buckle 69122 can slide along a lengthwise direction of the slot 69124 in the slot 69124, that is, the circumference of the skeleton 6912 is changeable within a certain range so that the supporting member 691 can adapt to various delivery sheaths 60 with various diameters.

### Ninth embodiment

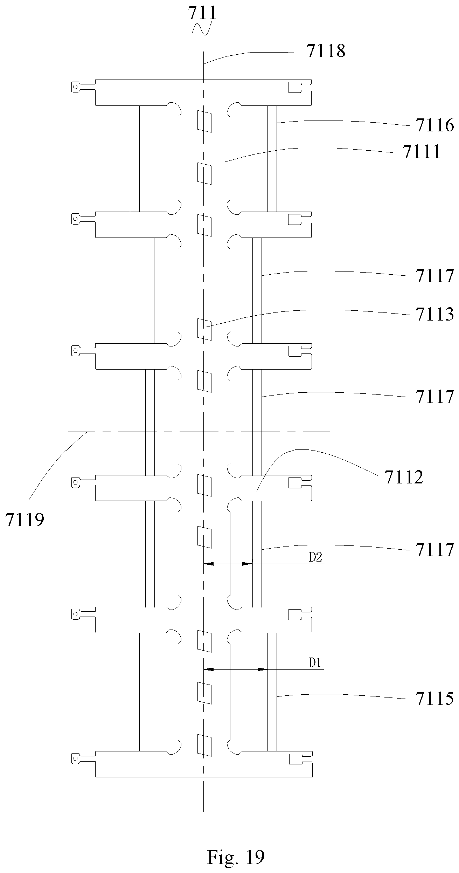

Referring to Fig. 19, a difference between the ninth embodiment and the eighth embodiment is that the supporting member 711 of this embodiment further includes a plurality of proximal reinforcing ribs 7115, distal reinforcing ribs 7116, and intermediate reinforcing ribs 7117. The proximal reinforcing ribs 7115, the distal reinforcing ribs 7116, and the intermediate reinforcing ribs 7117 are all arranged along a lengthwise direction of the keel 7111 in an extending manner, and the proximal reinforcing ribs 7115, the distal reinforcing ribs 7116, and the intermediate reinforcing ribs 7117 are arranged in pairs. A pair of proximal reinforcing ribs 7115 are provided between two adjacent skeletons 7112 at the proximal end of the supporting member 711, and this pair of proximal reinforcing ribs 7115 are symmetrical about a longitudinal central axis 7118 of the supporting member 711. A pair of distal reinforcing ribs 7116 are provided between two adjacent skeletons 7112 at the distal end of the supporting member 711, and this pair of distal reinforcing ribs 7116 are symmetrical about a longitudinal central axis 7118 of the supporting member 711. Along the lengthwise direction of the supporting member 711, the intermediate reinforcing ribs 7117 are arranged between two adjacent skeletons 7112 between the proximal reinforcing ribs 7115 and the distal reinforcing ribs 7116, and the intermediate reinforcing ribs 7117 are distributed in pairs on two sides of the longitudinal central axis 7118. The paired intermediate reinforcing ribs 7117 are symmetrical about the longitudinal central axis 7118. The proximal reinforcing ribs 7115, the distal reinforcing ribs 7116, and the intermediate reinforcing ribs 7117 can increase the axial supporting force of the supporting member, thus increasing the axial supporting force of the delivery sheath 60. The supporting member 711 is symmetrical about its transverse central axis 7119.

Along a width direction of the supporting member 711, the proximal reinforcing ribs 7115, the distal reinforcing ribs 7116, and the intermediate reinforcing ribs 7117 are all arranged apart from the keel 7111. For example, a distance from the proximal reinforcing rib 7115 to the longitudinal central axis 7118 is equal to the distance from the distal reinforcing rib 7116 to the longitudinal central axis 7118, and both are D1. The distance from the intermediate reinforcing rib 7117 to the longitudinal central axis 7118 is D2, and D1 is greater than or equal to D2.

In this embodiment, the proximal reinforcing ribs 7115, the distal reinforcing ribs 7116, and the intermediate reinforcing ribs 7117 are arranged apart from the keel 7111, and D1 is greater than or equal to D2, which increases the axial supporting force of the supporting member 711 and increases the compliance of the supporting member 711 during bending, thereby reducing the traction force required for the bending of the supporting member 711. The proximal reinforcing ribs 7115, the distal reinforcing ribs 7116 and the intermediate reinforcing ribs 7117 arranged in pairs are all symmetrical about the longitudinal central axis 7118, and the supporting member 711 is symmetrical about the transverse central axis 7119, which increases the axial supporting force of the supporting member 711 and increases the compliance of the supporting member 711 during bending, thereby reducing the traction force required for the bending of the supporting member 711.

It may be appreciated that, in other embodiments, the distance from the proximal reinforcing rib 7115 to the longitudinal central axis 7118 is not equal to the distance from the distal reinforcing rib 7116 to the longitudinal central axis 7118. The distance from the proximal reinforcing rib 7115 to the longitudinal central axis 7118 and the distance from the distal reinforcing rib 7116 to the longitudinal central axis 7118 are both greater than the distance from the intermediate reinforcing rib 7117 to the longitudinal central axis 7118, which can increase the axial supporting force of the supporting member 711 and increases the compliance of the supporting member 711 during bending, thereby reducing the traction force required for the bending of the supporting member 711.

### Tenth embodiment

This embodiment provides a medical device, including the delivery sheath in the above embodiments.

The features of the embodiments described above can be arbitrarily or deliberately combined. In order to make the description concise, all possible combinations of various features in the above embodiments are not completely described. However, the combinations of these features should be considered as the scope described in the embodiments as long as there is no contradiction in them.

The above-mentioned embodiments only express several implementation modes , and their descriptions are more specific and detailed, but they be understood as non-limiting of the scope of the embodiments. It should be noted that those of ordinary skill in the art can further make various transformations and improvements without departing from the concept of the embodiments, and these transformations and improvements all fall within the scope of the embodiments.

## Claims

1. A delivery sheath, comprising: a canal body portion, wherein that the canal body portion comprises:
an outer canal comprising a connecting canal, a canal body, and a distal canal, the canal body is located between the connecting canal and the distal canal; the canal body comprises a first flexible canal, a spacer canal, and a second flexible canal; the spacer canal is provided between a distal end of the first flexible canal and a proximal end of the second flexible canal; a hardness of the connecting canal is greater than that of the first and second flexible canals, and a hardness of the spacer canal is greater than that of the first and second flexible canals; and
a traction structure comprising a first traction fiber and a second traction fiber, the first traction fiber is connected to the first flexible canal, and the second traction fiber is connected to the second flexible canal; the first traction fiber and the second traction fiber both extend into the outer canal, and a free end of the first traction fiber and a free end of the second traction fiber extend out of a proximal end of the connecting canal; the first traction fiber may slide with respect to the canal body and may drive the first flexible canal to bend in a first plane; the second traction fiber may slide with respect to the canal body and may drives the second flexible canal to bend in a second plane; and the first plane intersects or is parallel to or is coplanar with the second plane.

2. The delivery sheath according to claim 1, wherein that the hardness of the first flexible canal is greater than or equal to that of the second flexible canal.

3. The delivery sheath according to claim 1, wherein that the canal body further comprises a transition canal;
the transition canal is arranged between the spacer canal and the first flexible canal; a hardness of the transition canal is less than that of the spacer canal; the hardness of the transition canal is greater than that of the first flexible canal;
or, the transition canal is arranged between the spacer canal and the second flexible canal; the hardness of the transition canal is less than that of the spacer canal; the hardness of the transition canal is greater than that of the second flexible canal;
or, the transition canal is arranged between the connecting canal and the first flexible canal; the hardness of the transition canal is less than that of the connecting canal; the hardness of the transition canal is greater than that of the first flexible canal;
or, the transition canal is arranged between the distal canal and the second flexible canal; the hardness of the transition canal is less than that of the distal canal; the hardness of the transition canal is greater than that of the second flexible canal.

4. The delivery sheath according to claim 1, wherein that the first plane is perpendicular to the second plane.

5. The delivery sheath according to claim 1, wherein the traction structure further comprises a first fixed structure and/or a second fixed structure fixed in the canal body; the first fixed structure is arranged at the distal end of the first flexible canal; the second fixed structure is arranged at the distal end of the second flexible canal; a first connecting hole, a second connecting hole, and a third connecting hole are formed in a side wall of the first fixed structure and/or the second fixed structure; along a circumferential direction of the first fixed structure and/or the second fixed structure, the second connecting hole is located between the first connecting hole and the third connecting hole; connecting lines of hole centers of the first connecting hole, the second connecting hole, and the third connecting hole may form a triangle; an inner angle formed by the triangle at the hole center of the second connecting hole is an obtuse angle; the first traction fiber passes through the first connecting hole, the second connecting hole, and the third connecting hole in sequence to be connected with the first fixed structure; and/or, the second traction fiber passes through the first connecting hole, the second connecting hole, and the third connecting hole of the second fixed structure in sequence to be connected to the second fixed structure.

6. The delivery sheath according to claim 1, wherein the canal body further comprises at least one supporting member arranged in the canal body; when there is only one supporting member, the supporting member is arranged in a section where the first flexible canal or the second flexible canal is located; and when there are a plurality of supporting members, the plurality of supporting members are arranged in sections where the first flexible canal and the second flexible canal are located.

7. The delivery sheath according to claim 6, wherein the supporting member comprises an extension body that extends spirally; after one side wall of the supporting member is cut along a direction parallel to a central axis of the supporting member, the cut supporting member is spread along a radial direction of the supporting member; and an included angle formed by the extension body and the central axis of the supporting member ranges from 60 degrees to 90 degrees.

8. The delivery sheath according to claim 1, wherein that the first flexible canal is bent in a natural state; and an included angle formed between an extending direction of a proximal end part of the first flexible canal and an extending direction of a distal end part of the first flexible canal ranges from 45 degrees to 90 degrees.

9. The delivery sheath according to claim 1, wherein the spacer canal is linear or bent in a natural state of the spacer canal; when the spacer canal is bent in the natural state, the spacer canal is bent in the second plane; a bending direction of the spacer canal is opposite to a bending direction of the second flexible canal; and when the spacer canal is linear in the natural state, the spacer canal is linear in the second plane.

10. The delivery sheath according to claim 6, wherein the supporting member comprises a keel and a plurality of skeletons; the plurality of skeletons are arranged apart from each other along an axial direction of the keel; two ends of the plurality of skeletons are respectively connected to two sides of the keel; the keel and the plurality of skeletons together form an accommodating space; and the canal wall of the delivery sheath is accommodated in the accommodating space.

11. The delivery sheath according to claim 10, wherein a most distal skeleton of the plurality of skeletons has a distal end surface; a distal end part of the keel is flush with the distal end surface, or the distal end part of the keel is located on a proximal side of the distal end surface.

12. The delivery sheath according to claim 10, wherein a most proximal skeleton of the plurality of skeletons has a proximal end surface; a proximal end part of the keel is flush with the proximal end surface, or the proximal end part of the keel is located on a distal side of the proximal end surface.

13. The delivery sheath according to claim 10, wherein the plurality of skeletons each comprise a first connecting part and a second connecting part; the first connecting part and the second connecting part are oppositely arranged on two sides of the keel; a free end of the first connecting part is provided with a buckle, and a free end of the second connecting part is provided with a slot; and the buckle and the slot are snap-fitted.

14. The delivery sheath according to claim 13, wherein the slot is elastic, and a slot width of the slot may increase under the action of an external force on the slot.

15. The delivery sheath according to claim 10, wherein the keel is further provided with a hollow structure.

16. The delivery sheath according to claim 10, wherein the supporting member further comprises a proximal reinforcing rib, a distal reinforcing rib, and an intermediate reinforcing rib; the proximal reinforcing rib, the distal reinforcing rib, and the intermediate reinforcing rib are all arranged along a lengthwise direction of the keel in an extending manner; the distal reinforcing rib is arranged between two adjacent skeletons at the distal end of the supporting member; the proximal reinforcing rib is arranged between two adjacent skeletons at the proximal end of the supporting member; and the intermediate reinforcing rib is arranged between two adjacent skeletons between the proximal reinforcing rib and the distal reinforcing rib.

17. The delivery sheath according to claim 16, wherein the proximal reinforcing rib, the distal reinforcing rib, and the intermediate reinforcing rib are all arranged apart from the keel; a distance from the proximal reinforcing rib to a longitudinal central axis of the supporting member is greater than a distance from the intermediate reinforcing rib to the longitudinal central axis of the supporting member; and a distance from the distal reinforcing rib to the longitudinal central axis of the supporting member is greater than the distance from the intermediate reinforcing rib to the longitudinal central axis of the supporting member.

18. The delivery sheath according to claim 6, wherein the supporting member comprises a plurality of hollow sub-components connected end to end; a proximal end of each hollow sub-component comprises a plurality of proximal protrusions distributed along a circumferential direction of the hollow sub-component; a proximal groove is formed between two adjacent proximal protrusions; a distal end of each hollow sub-component comprises a plurality of distal protrusions distributed along the circumferential direction of the hollow sub-component; a distal groove is formed between two adjacent distal protrusions; the number of the proximal protrusions of the hollow sub-component is equal to the number of the distal protrusions of the same hollow sub-component; one distal groove on the hollow sub-component is aligned with one proximal protrusion on the same hollow sub-component; and the plurality of proximal protrusions on one hollow sub-component are meshed with the plurality of distal grooves on the other hollow sub-component.

19. The delivery sheath according to claim 18, wherein a bevel edge of the proximal protrusion connected to the proximal end and the distal end of the proximal protrusion is provided with a proximal bulge, and/or a bevel edge of the distal protrusion connected to the proximal end and the distal end of the distal protrusion is provided with a distal recess.

20. A medical device, wherein that the medical device comprises the delivery sheath according to of claim 1.
